(19) Europäisches Patentamt
European Patent Office
Office européen des brevets

(11) EP 1 278 531 B1

(12) EUROPÄISCHE PATENTSCHRIFT

(45) Veröffentlichungstag und Bekanntmachung des Hinweises auf die Patenterteilung:
**21.09.2005 Patentblatt 2005/38**

(21) Anmeldenummer: **01921095.4**

(22) Anmeldetag: **25.04.2001**

(51) Int Cl.[7]: **A61K 35/78**, A61P 31/22

(86) Internationale Anmeldenummer:
**PCT/CH2001/000257**

(87) Internationale Veröffentlichungsnummer:
**WO 2001/085189 (15.11.2001 Gazette 2001/46)**

# (54) VERWENDUNG EINER ZUBEREITUNG ZUR TOPISCHEN BEHANDLUNG VON DURCH VIRUSINFEKTIONEN VERURSACHTEN MANIFESTATIONEN

USE OF A PREPARATION FOR TOPICALLY TREATING MANIFESTATIONS CAUSED BY VIRUS INFECTIONS

UTILISTION D'UNE PREPARATION POUR TRAITEMENT TOPIQUE DE MANIFESTATIONS INDUITES PAR INFECTIONS VIRALES

(84) Benannte Vertragsstaaten:
**AT CH DE LI**

(30) Priorität: **05.05.2000 CH 887002000**

(43) Veröffentlichungstag der Anmeldung:
**29.01.2003 Patentblatt 2003/05**

(73) Patentinhaber: **Parsenn-Produkte AG**
**7240 Küblis (CH)**

(72) Erfinder: **LÜSCHER, Erich**
**CH-7247 Saas (CH)**

(74) Vertreter: **Schaad, Balass, Menzl & Partner AG**
**Dufourstrasse 101**
**Postfach**
**8034 Zürich (CH)**

(56) Entgegenhaltungen:
**WO-A-99/66942**

- **DATABASE WPI Section Ch, Week 200140 Derwent Publications Ltd., London, GB; Class B04, AN 2001-375254 XP002176839 & CN 1 157 160 A (WANG H), 20. August 1997 (1997-08-20)**
- **DATABASE BIOSIS [Online] BIOSCIENCES INFORMATION SERVICE, PHILADELPHIA, PA, US; 1989 SYDISKIS R J ET AL: "INACTIVATION OF HERPES SIMPLEX VIRUS BY ANTHRAQUINONES ISOLATED FROM PLANTS" Database accession no. PREV198937071384 XP002176838 & JOURNAL OF DENTAL RESEARCH, Bd. 68, Nr. SPEC. ISSUE JUNE, 1989, Seite 935 ISSN: 0022-0345**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die Verwendung einer Zubereitung zur Herstellung eines Arzneimittels zur topischen Behandlung von durch Virusinfektionen verursachten Manifestationen.

Infektionen mit dem Herpes Simplex Virus (HSV) 1 oder 2 gehören zu den häufigsten viralen Erkrankungen bei Menschen. Eine fundamentale biologische Eigenheit der meisten Herpes-Viren (Herpes Simplex (HSV) 1 und 2, Varicella Zoster (VZV), Cytomegalovirus (CMV) und Epstein-Barr (EBV)) ist ihre Fähigkeit im Körper des Wirtes latent zu persistieren. Eine klinische Reaktivierung der EBV, CMV und VZV wird selten beobachtet, dagegen kommt sie bei den HSV häufig vor. Ein Rezidiv kann durch intensive Sonnenbestrahlung, Fieber, hormonelle Einflüsse oder Schwächung der Immunabwehr ausgelöst werden.

Bei orofacialen rekurrenten Infektionen sind Fieberblasen die häufigste Manifestation. Rund 2/3 der Patienten weisen prodromale Symptome wie Schmerz oder Juckreiz an der Stelle der nachfolgenden Fieberblase oder Läsion auf. Innerhalb weniger Stunden folgen auf das Prodrom kleine erythemaöse Knötchen, die schnell zu intradermalen Vesikeln auf dem Lippenrand oder der angrenzenden Gesichtshaut führen. Die meisten Vesikel bilden innerhalb von 24 h Geschwüre oder Läsionen aus, die dann schnell austrocknen und verkrusten.

Die Patienten suchen aufgrund der Schmerzen, der Entstellungen und der psychologischen Belastung eine schnell wirkende und effektive Therapie. Die Befriedigung dieser Bedürfnisse wurde durch eine Vielzahl verschiedener Wirkstoffe versucht. Beispiele dafür sind systemische Behandlungen mit Idoxuridin (2'-Deoxy-5-iodouridin), Cytosinarabinosid (4-Amino-1-β-D-arabinofuranosyl-2(1*H*)-pyrimidon), Adeninarabinosid (9-β-D-Arabinofuranosyl-9*H*-purin-6-amin), Interferon sowie die Immunisierung mit einem Herpes Simplex Vakzin, aber auch topische Behandlungen mit Idoxuridin in Dimethylsulphoxid, Phosphonessigsäure, Ether sowie Acylovir (9-[(2-Hydroxyethoxy)methyl]guanin; Zovirax®). Für eine schnelle Behandlung der Läsionen und Erytheme hat sich Acyclovir besonders gut bewährt.

Die topische Anwendung des synthetisch hergestellten Acyclovir weist den bisher bekannten Therapieformen gegenüber einige Vorteile auf. Nebst seiner Effizienz ist es gut verträglich, da sein Wirkungsmechanismus sehr spezifisch gegen das Herpes Simplex Virus gerichtet ist. Die Thymidinkinase, die durch das HSV codiert ist, führt Acyclovir in Acyclovirmonophosphat über, das in ein Diphosphat und schliesslich durch Zellenzyme ins Triphosphat umgewandelt wird. Durch den Einbau des Acyclovirs in die Virus-DNA wird diese unterbrochen. Mithin wird eine virale DNA-Replikation verhindert. Die Thymidinkinase der normalen, nicht infizierten Zelle verwendet Acyclovir kaum als Substrat, daher ist die Toxizität auf die Wirtszelle sehr gering.

Allerdings haben viele Patienten haben eine Abneigung gegen einen Eingriff ins genetische Material und lehnen deshalb entsprechende Substanzen ab. Weiter wurde festgestellt, dass langdauernde und wiederholte Anwendungen von Acyclovir bei Schwerimmunsupprimierten zu Virusstämmen mit reduzierter Empfindlichkeit führen können, die nicht mehr auf eine Acyclovirtherapie ansprechen. Ein letzter hier aufgeführter Nachteil des Acyclovirs ist die potentielle Gefahr der Erzeugung veränderter Viren.

Der Rhabarber ist für die laxative und adstringierende Wirkung der darin vorkommenden Gerbstoffe bekannt. Der adstringierenden Wirkung der Gerbstoffe liegt ihre Bindung an bestimmte kolloidale Strukturen zugrunde, die zu einer Abnahme des Dispersionsgrades und damit zu einer Verdichtung der betroffenen Gewebestelle führt. Gerbstoffe werden von der Haut und von Schleimhäuten kaum resorbiert, sie können daher im allgemeinen nur örtliche, aber keine resorptiven Wirkungen ausüben. Rhabarberextrakte haben aufgrund ihrer Gerbwirkung beispielsweise bei Affektionen der Mundschleimhaut und des Zahnfleisches sowie bei Aphten eine Anwendung gefunden (Pyralvex®).

In der Literatur wurde gezeigt, dass Substanzen, die aus den Blättern von Aloe-Pflanzen extrahiert wurden, in vitro (Vero- und WI-38-Zellkulturen) eine antivirale Aktivität aufweisen (Saoo, M.; Miki, H.; Ohmori, M. (1996) *Phytotherapy Research,* 10, 348-350). Eine analoge Wirkung konnten Sydiskis und Mitarbeiter bei Extrakten der Rhabarberwurzeln in MRC-5-Zellkulturen feststellen (Sydiskis, R.J.; Owen, D.G.; Lohr, J.L.; Tosler, K-H.; Blomster, R.N. (1991) *Antimicrobial Agents and Chemotherapy,* 2463-2466). Aufgrund dieser Literatur wurden mit einer Rhabarberextrakt-Creme in vivo Versuche mit rekurrent erkrankten HSV-Patienten durchgeführt, die jedoch nicht zu den gewünschten Ergebnissen geführt haben.

Die heilende Wirkung, die Salbei erzielen kann, ist seit dem Altertum bekannt. Der häufigste Einsatz von Salbei erfolgt bei entzündlichen Prozessen im Mund, Rachen sowie am Zahnfleisch. Bei Gingivitis, Stomatitis, Laryngitis und Pharngitis werden schon seit langem Salbeiabkochungen zum Gurgeln empfohlen. Verschiedene Salbeienthaltende Tropfen sind bekannt, die den Heilungsprozess der Entzündungen fördern. Ebenso ist das Kauen von Salbeiblättern oder Lutschen von Salbei-Bonbons bei oben erwähnten Entzündungen gesundheitsfördernd. Ausserdem ist Salbei auch als auswurffördernes Phytotherapeuikum anerkannt. Expektorantia, die Salbei enthalten, werden häufig als Tropfen oder Dragees verabreicht.

Einem Inhaltsstoff des Salbeiblattextraktes, der Rosmarinsäure (1),

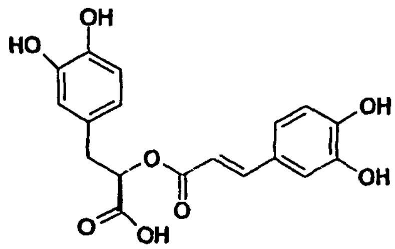

(1)

wird eine besondere Bedeutung beigemessen. Dabei handelt es sich um einen schwach adstringierenden Gerbstoff, dem ausserdem eine entzündungshemmende und antivirale Wirkung nachgewiesen werden konnte. Die entzündungshemmende Wirkung der Rosmarinsäure beruht vermutlich auf ihrer Fähigkeit die Aktivität der C3-Konvertase des klassischen und des alternativen Weges des Komplementsystems zu beeinträchtigen. Eine Aktivierung des Komplementsystems durch einen Stimulus löst unter anderem die Arachidonsäurekaskade aus. Mithin kann die Rosmarinsäure durch Inhibierung der C3-Konvertase die Prostaglandinsynthese unterdrücken. Die Regulierung der Komplementaktivität durch die Rosmarinsäure hat gegenüber den NSAID (nonsteroidal anti-inflammatory drugs) den Vorteil, dass die Inhibierung der Prostaglandinausschüttung spezifisch auf die Entzündungsstelle begrenzt ist, bei der die Komplementaktivierung auftritt. Im Gegensatz dazu können bei der Verwendung der NSAIDs oder der Glucocortinoide infolge der Inhibierung der Eicosanoid-Bildung im ganzen Körper (Magen, Niere, Blutplättchen) unerwünschte Nebenwirkungen auftreten. (Rampert, M.; Beetens, H.R.; Bult, H.; Herman, A.G.; Parnham, M.J.; Winkelmann, J. (1986) *Biochemical Pharmacology,* 35, No. 8, 1397-1400). Über den genauen Mechanismus der antiviralen Wirkung der Rosmarinsäure ist zum jetzigen Zeitpunkt noch wenig bekannt. Aufgrund dieser in der Literatur beschriebenen positiven in vitro Resultate wurden mit einer Salbei-Creme *in vivo* Versuche mit rekurrent erkrankten HSV-Patienten durchgeführt. Dabei hat die Salbeiblatt-Creme jedoch deutlich schlechter abgeschnitten als das Kontrollmedikament Zovirax®.

Die durch die Virusinfektionen verursachten Manifestationen sind Erytheme, Knötchen, Fieberblasen, Läsionen und Geschwüre. In der vorliegenden Erfindung wird eine Zubereitung vorgeschlagen, die eine kombinierte antivirale und heilungsfördernde Wirkung hat. Durch die kombinierte Verwendung von Rhabarberextrakt und Salbeiextrakt konnte überraschenderweise eine synergistische Wirkung erreicht werden. Die jeweiligen Extrakte haben zwar - wie die Überprüfung gezeigt hat - *in vivo* eine geringfügige Wirkung, die jedoch keinen Anlass zu weiteren Versuchen gab, da sie zur Begründung eines Heilungsprozesses nicht ausreichen. Die Kombination der beiden Extrakte dagegen wirkt gleich schnell wie das Kontrollmedikament Zovirax® und stellt daher eine gute Behandlungsalternative auf natürlicher Basis dar.

Die Zubereitung auf natürlicher Basis kann zudem Patienten dienen, die schon eine Vielzahl anderer Medikamente zu sich nehmen müssen, wie zum Beispiel schwerimmunsupprimierte (HIV-Positive und Transplantationspatienten) Herpes-Patienten.

Vorzugsweise ist der Rhabarberextrakt, der in der vorliegenden Erfindung enthalten ist, ein Rhabarberwurzelextrakt.

Für die erfindungsgemässe Zubereitung werden Rhabarberwurzelextrakte bevorzugt, die mindestens 4% Hydroxyanthracen-Derivate, berechnet als Rhein (9,10-Dihydro-4,5-dihydroxy-9,10-dioxo-2-anthracencarbonsäure) und bezogen auf den getrockneten Extrakt, enthalten.

Für die erfindungsgemässe Zubereitung werden Rhabarberwurzelextrakte bevorzugt, die Emodin (R=OH) und/oder Aloe-Emodin (R=$CH_2OH$) der Formel (2)

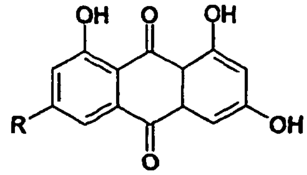

(2)

enthalten.

Vorzugsweise ist der Salbeiextrakt, der in der vorliegenden Erfindung enthalten ist, ein Salbeiblattextrakt.

Für die erfindungsgemässe Zubereitung werden Salbeiblattextrakte vorgezogen, die mindestens 1.5% Gerbstoffe, berechnet als Pyrogallol und bezogen auf die Trockensubstanz, enthalten.

Insbesondere werden für die erfindungsgemässe Zubereitung Salbeiblattextrakte, die Rosmarinsäure (2-[3-(3,4-dihydroxyphenyl-1-oxo-2-propinyl]-3-(3,4-dihydroxyphenyl)-propionsäure) enthalten.

Die erfindungsgemässe Zubereitung wird vor allem zur topischen Behandlung von Manifestationen die durch umhüllte Viren verursacht worden sind. Die erfindungsgemässe Zubereitung wird vorzugsweise zur topischen Behandlung von durch Herpes Virusinfektionen verursachten Manifestationen angewendet. Insbesondere wird die erfindungsgemässe Zubereitung zur topischen Behandlung von durch Herpes Simplex Virusinfektionen 1 und 2 verursachten Manifestationen eingesetzt.

Die erfindungsgemässe Zubereitung kann durch eine Vielzahl verschiedener Darreichungsformen verabreicht werden, das heisst sie liegt als Salbe, Creme, Paste, Gel, Lotion, Stift oder Tinktur vor. Vorzugsweise ist die erfindungsgemäse Zubereitung eine Creme oder ein Stift. Die weiche Konsistenz der erfindungsgemässen Zubereitung wirkt sich ausnehmend vorteilhaft aus.

Die in der erfindungsgemässen Zubereitung enthaltenen Rhabarber- und Salbeiextrakte sind vorzugsweise im Mischungsverhältnis 1:1.

Die erfindungsgemässe Zubereitung wird durch Zusammenbringen und Vermischen einer Ölphase, einer Wasserphase, der Emulgatoren sowie der Salbei- und Rhabarberextrakte hergestellt, wobei vorzugsweise die Zugabe des Rhabarberextraktes und des Salbeiextraktes vor derjenigen der Wasserphase erfolgt.

Die Kombination von Rhabarberextrakt und Salbeiextrakt, die in der vorliegenden Erfindung enthalten sind, werden vorzugsweise als Mittel zur topischen Behandlung von durch Virusinfektionen verursachten Manifestationen verwendet.

Die Wirkstoffe Rosmarinsäure, Emodin sowie Aloe-Emodin der erfindungsgemässen Zubereitung könnten auch aus anderen. Pflanzen oder Pilzen extrahiert werden. Nicht beschränkende Beispiele hierfür sind Flechten, Sauerampfer, Aloe sowie Melisse.

Die nachstehenden Beispiele verdeutlichen die vorliegende Erfindung ohne diese einzuschränken.

Die folgenden Chemikalien sind kommerziell erhältlich: 4-Hydroxybenzoesäuremethylester, 4-Hydroxybenzoesäurepropylester, Propylenglykol, Glycerol, Natriumdodecylsulfat, Cetylstearylalkohol, Wollwachs, Isopropylmyristat, Lactose-Monohydrat, hochdisperses Siliciumdioxid.

**Beispiel 1: Rhabarberextrakt**

**Herstellung** (Lit. DAB 10, 2. Nachtrag, 1993)

Geschnittene Rhabarberwurzeln werden mit 70% Ethanol (V/V) nach dem Verfahren der Perkolation ausgezogen. Die Perkolation ist beendet, wenn 3 Teile Perkolat abgetropft sind. Das mit der Pressflüssigkeit vereinigte Perkolat wird nach 24 h langem Stehenlassen durch Watte filtriert und zur Trockene eingedampft. Der Extrakt wird pulverisiert und im Exsikkator nachgetrocknet. Der Gehalt an Hydroxyanthracen-Deriväten wird bestimmt und der Extrakt gegebenenfalls durch Verreiben mit Lactose oder Dextrin auf den geforderten Gehalt nach der Gleichung

$$m_1 = \frac{m_2 \cdot (a-5)}{5}$$

eingestellt, wobei $m_1$ dem Gewicht der Lactose oder des Dextrins in Gramm, $m_2$ dem Gewicht des einzustellenden Extraktes in Gramm und a dem Gehalt des Extraktes an Hydroxyanthracen-Derivaten in Prozent entspricht.

Der so erhaltene Extrakt ist eine braune, hygroskopische, pulverförmige oder pulversierbare Masse von eigenartigem Geruch und bitterem Geschmack, die wenig löslich in Wasser, jedoch gut löslich in 70% Ethanol ist.

**Charakterisierung:**

Die Charakterisierung erfolgt mittels Dünnschichtchromatographie. Als stationäre Phase wird eine Merck-DC-Glasplatte, Kieselgel 60 F254, 20x20 cm verwendet. Die mobile Phase besteht aus Ethylacetat/Methanol/Wasser (100/13.5/10) und als Visualisierungsmittel wird 5% (m/V) Kaliumhydroxid in Ethanol benutzt.

| **Substanz** | **$R_f$** | **rel. $R_f$*(0.20)** | **Farbe** |
|---|---|---|---|
| Aloe-Emodin | 0.94 | 1 | rot-braun |
| Emodin | 0.92 | 0.98 | orange-braun |

* Der relative $R_f$ bezieht sich auf die Referenzsubstanz Aloe-Emodin

Der so eingestellte Rhabarberextrakt (Rhei extractum siccum normatum) enthält mindestens 4.0 und höchstens 6.0 % Hydroxyanthracen-Deriwte, berechnet als Rhein ($C_{15}H_8O_6$) und bezogen auf den getrockneten Extrakt.
Ein Rhabarberextrakt der oben genannten Qualität kann zum Beispiel bei Extrakt Chemie, Stadthagen (Deutschland) erworben werden.

**Beispiel 2: Salbeiextrakt**

**Herstellung**

400 kg geschnittene Salbeiblätter werden mit Wasser bei 75-85°C nach dem Verfahren der Perkloration erschöpfend ausgezogen. Das Perklorat wird bis zum Spissumextrakt eingedampft. 80 kg des so erhaltenen Spissumextrakt werden mit 17 kg Lactose-Monohydrat und 3 kg hochdispersem Siliciumoxid versetzt und getrocknet. Der Trockenextrakt wird gemahlen und gemischt.
Der so erhaltene bräunliche bis braune Extrakt hat einen charakteristischen Geruch.

**Charakterisierung:**

Die Charakterisierung erfolgt mittels Dünnschichtchromatographie. Als stationäre Phase wird eine Merck-DC-Glasplatte, Kieselgel 60 F254, 20x20 cm verwendet. Die mobile Phase besteht aus Toluol/Ameisensäureethylester/Ameisensäure/Wasser (5/100/10/10). Zur Visualisierung wird die Platte mit 1% Naturstoffreagenz in Methanol versetzt und nach 10-minütigem Trocknen mit 5% Polyethylenglykol 4000 in Ethanol besprüht.

| Substanz | $R_f$ | Farbe |
|---|---|---|
| Rosmarinsäure | 0.82 | blau-fluoreszierend |

Der so eingestellte Salbeiblattextrakt enthält mindestens 1.5 % Gerbstoffe, berechnet als Pyrogallol bezogen auf die Trockensubstanz.
Ein Salbeiblattextrakt der oben genannten Qualität kann zum Beispiel bei H. Finzelberg's Nachf. GmbH & Co KG, Andernach (Deutschland)erworben werden.

**Beispiel 3: Klinische Studie**

Zur Dokumentation der Wirksamkeit und Verträglichkeit einer Creme auf der Basis von Rhabarber- und Salbeiextrakten gegen orofaciale Herpes Simplex Virusinfektionen wurde eine randomisierte Doppelblindstudie ausgeführt. Als primäre Wirksamkeitskriterien wurde die Dauer bis zum vollständigen Verschwinden der Läsion, die Dauer bis zur Abtrocknung und/oder zum Beginn der Krustenbildung sowie der klinische Verlauf der Symptome festgelegt. Die sekundären Wirksamkeitskriterien bestanden aus Dauer bis zum nächsten Rezidiv, den Therapieversagern (Weiterbestehen der Läsion am Tag 10-14), das Auftreten einer Komplikation (Ausdehnung oder Streuung der Läsion oder eine Superinfektion am Tag 10-14) sowie ein Urteil zur Verträglichkeit und Wirksamkeit.
Als Prüfmedikament diente eine Rhabarber-Salbeicreme [23 mg Rhabarberextrakt und 23 mg Salbeiextrakt pro Gramm] und als Kontrollmedikament die Zoviraxcreme® [50 mg Acyclovir pro Gramm]. Die Zoviraxcreme® war zum Zeitpunkt der klinischen Studie (1994) das beste auf dem Markt befindliche Präparat. Das Dosierungsschema orientierte sich an demjenigen der Zoviraxcreme® (5-mal täglich in einem ungefähr 4-Stunden-Intervall).
Die Resultate der Studie haben gezeigt, dass kein signifikanter Unterschied zwischen Zovirax® und der Rhabarber-Salbeicreme vorliegt. Der Mittelwert der Heilungszeit aller geheilten Patienten betrug bei der Zovirax®-Gruppe (N=38) 6.53 Tage (Standardabweichung 2.88 Tage), bei der Rhabarber-Salbei-Gruppe (N=57) 6.68 Tage (Standardabweichung 2.7 Tage). Die Resultate der Studie ergeben für keines der getesteten Präparate einen Hinweis auf ein Risiko.
Daraus lässt sich die Schlussfolgerung ziehen, dass die Rhabarber-Salbeicreme sich ebenso zur Behandlung von orofacialen Herpes Simplex Virusinfektionen eignet, wie die Zoviraxcreme®.

**Beispiel 4: Galenische Formulierung**

Die nachstehende Tabelle 1 zeigt eine mögliche Zusammensetzung einer erfindungsgemässen Zubereitung auf einer Cremen-Basis.

Tabelle 1:

| Zusammensetzung des Arzneimittels: | | | |
|---|---|---|---|
| Eingestellter Rhabarbertrockenextrakt | 2.3% | DAB | Wirkstoff |
| Trockenextrakt aus Salbeiblättern | 1.84% | Finzelberg | Wirkstoff |
| 4-Hydroxybenzoesäuremethylester | 0.3% | Ph.Eur. | Konservierungsmitt el |
| 4-Hydroxybenzoesäurepropylester | 0.2% | Ph.Eur. | Konservierungsmitt el |
| Propylenglykol | 30% | Ph.Eur. | Lösungsmittel |
| Glycerol 85% | 9% | Ph.Eur. | Feuchthaltemittel |
| gereinigtes Wasser | 37.3% | Ph.Eur. | äussere Phase der Emulsion, Lösungsmittel für die Extrakte |
| Natrimdodecylsulfat | 1.3% | Ph.Eur. | Emulgator |
| Cetylstearylalkohol | 11.7% | Ph.Eur. | Emulgator |
| Wollwachs | 0.6% | Ph.Eur. | Emulgator |
| Isopropymyristat | 5% | Ph.Eur. | Emulgator |
| Lactose-Monohydrat | 0.39% | Ph.Eur. | Füllmittel für die Salbeiextraktzubereitung |
| Hochdisperses Siliciumdioxid | 0.07% | Ph.Eur. | Fliessmittel |
| | 100% | | |

**Herstellung:**

26 kg Propylenglycol und 9 kg Glycerin werden in der Prozessanlage vorgelegt. 2.3 kg Rhabarber- und 2.3 kg Salbeiextrakt werden unter Rühren beigegeben. 1.3 kg Natriumdodecylsulfat werden in 37.3 1 Wasser gelöst und unter Rühren der oben hergestellten Lösung zugegeben. Es entsteht eine klare braune Lösung. Diese wird unter Rühren auf 70-75°C geheizt.

11.7 kg Cetylstearylalkohol, 0.6 kg Wollwachs und 5 kg Isopropylmyristat werden auf 70-75°C unter Rühren erwärmt bis eine klare Schmelze entsteht. Diese wird der klaren dunkelbraunen Lösung unter schnellem Rühren beigegeben.

In 4 kg Propylenglykol werden 0.3 kg 4-Hydroxybenzoesäuremethylester und 0.2 kg 4-Hydroxybenzoesäurepropylester gegeben und gelöst. Die so entstandene klare Lösung wird dem Ansatz beigegeben und 12-18 Minuten weiter gerührt. Anschliessend wird die Suspension unter Rühren gekühlt bis sie eine Temperatur von 40-45°C erreicht. Nach einer 7-10 Minuten dauernden Homogenisierung wird die so entstandene dunkelbraune Creme unter Rühren auf Raumtemperatur abgekühlt.

**Beispiele 5-14: Wirkstoff-Konzentrationen**

Die Creme wird analog zu der in Beispiel 4 hergestellt, jedoch werden die Mischungsverhältnisse der Extrakte wie folgt verändert (alle Angaben sind in %):

| Beispiel | 5 | 6 | 7 | 8 | 9 | 10 | 11 | 12 | 13 | 14 |
|---|---|---|---|---|---|---|---|---|---|---|
| Rhabarber-Trockenextrakt | 1.53 | 1.15 | 0.92 | 3.07 | 3.45 | 3.68 | 2.7 | 3.2 | 2.2 | 2.2 |
| Trockenextrakt aus Salbeiblättern + Lactose Monohydrat + hochdisperses Siliciumoxid | 3.07 | 3.45 | 3.68 | 1.53 | 1.15 | 0.92 | 2.2 | 2.2 | 2.7 | 3.5 |
| Total Extrakt | 4.6 | 4.6 | 4.6 | 4.6 | 4.6 | 4.6 | 4.9 | 5.4 | 4.9 | 5.4 |

**Patentansprüche**

1. Verwendung einer Kombination von einem Rhabarberextrakt und einem Salbeiextrakt zur Herstellung eines Mittels

zur topischen Behandlung von durch virusinfektionen verursachten Manifestationen.

2. Verwendung gemäss Anspruch 1 **dadurch gekennzeichnet, dass** der in der Kombination enthaltene Rhabarberextrakt ein Rhabarberwurzelextrakt ist.

3. Verwendung gemäss einem der Ansprüche 1 und 2 **dadurch gekennzeichnet, dass** der in der Kombination enthaltene Rhabarberwurzelextrakt mindestens 4% Hydroxyanthracen-Derivate, bezogen auf den getrockneten Extrakt, enthält.

4. Verwendung gemäss einem der Ansprüche 1 bis 3 **dadurch gekennzeichnet, dass** die Hydroxyanthracen-Derivate des in der Kombination enthaltenen Rhabarberwurzelextraktes Emodin und/oder Aloe-Emodin enthalten.

5. Verwendung gemäss einem der Ansprüche 1 bis 4, **dadurch gekennzeichnet, dass** der in der Kombination enthaltene Salbeiextrakt ein salbeiblattextrakt ist.

6. Verwendung gemäss einem der Ansprüche 1 bis 5 **dadurch gekennzeichnet, dass** der in der Kombination enthaltene Salbeiblattextrakt mindestens 1.5% Gerbstoffe, bezogen auf die Trockensubstanz, enthält.

7. Verwendung gemäss einem der Ansprüche 1 bis 6 **dadurch gekennzeichnet, dass** die Gerbstoffe des in der Kombination enthaltenen Salbeiblattextraktes Rosmarinsäure enthalten.

8. Verwendung gemäss einem der Ansprüche 1 bis 7 zur topischen Behandlung von Manifestationen, die durch umhüllte Viren verursacht worden sind.

9. Verwendung gemäss einem der Ansprüche 1 bis 8 zur topischen Behandlung von durch Herpes Virusinfektionen verursachten Manifestationen.

10. Verwendung gemäss einem der Ansprüche 1 bis 9 zur topischen Behandlung von durch Herpes Simplex Virusinfektionen 1 und 2 verursachten Manifestationen.

11. Verwendung gemäss einem der Ansprüche 1 bis 10 wobei das Mittel in Form einer Creme oder eines Stiftes vorliegt.

12. Verwendung gemäss einem der Ansprüche 1 bis 11 **dadurch gekennzeichnet, dass** die in der kombination enthaltenen Extrakte in einem Mischungsverhältnis 1:1 enthalten sind.

## Claims

1. Use of a combination of a rhubarb extract and a sage extract for preparing a remedy for the topical treatment of manifestations caused by viral infections.

2. Use according to Claim 1, **characterized in that** the rhubarb extract present in the combination is a rhubarb root extract.

3. Use according to either of Claims 1 and 2, **characterized in that** the rhubarb root extract present in the combination comprises at least 4% hydroxyanthracene derivatives, based on the dried extract.

4. Use according to any of Claims 1 to 3, **characterized in that** the hydroxyanthracene derivatives of the rhubarb root extract present in the combination comprise emodin and/or aloe-emodin.

5. Use according to any of Claims 1 to 4, **characterized in that** the sage extract present in the combination is a sage leaf extract.

6. Use according to any of Claims 1 to 5, **characterized in that** the sage leaf extract present in the combination comprises at least 1.5% tannins, based on dry substance.

7. Use according to any of Claims 1 to 6, **characterized in that** the tannins of the sage leaf extract present in the

combination comprise rosmarinic acid.

8. Use according to any of Claims 1 to 7 for the topical treatment of manifestations caused by enveloped viruses.

9. Use according to any of Claims 1 to 8 for the topical treatment of manifestations caused by herpes virus infections.

10. Use according to any of Claims 1 to 9 for the topical treatment of manifestations caused by herpes simplex virus type-1 and type-2 infections.

11. Use according to any of Claims 1 to 10, the remedy being in the form of a cream or a stick.

12. Use according to any of Claims 1 to 11, **characterized in that** the extracts are present in a 1:1 mixture in the combination.

**Revendications**

1. Utilisation d'une association d'un extrait de rhubarbe et d'un extrait de sauge pour la préparation d'une composition destinée au traitement local de manifestations provoquées par des infections virales.

2. Utilisation selon la revendication 1, **caractérisée en ce que** l'extrait de rhubarbe contenu dans l'association est un extrait de racines de rhubarbe.

3. Utilisation selon la revendication 1 ou 2, **caractérisée en ce que** l'extrait de racines de rhubarbe contenu dans l'association contient au moins 4 % de dérivés d'hydroxyanthracène, par rapport à l'extrait séché.

4. Utilisation selon l'une quelconque des revendication 1 à 3, **caractérisée en ce que** les dérivés d'hydroxyanthracène de l'extrait contenu dans l'association contiennent de l'émodine et/ou de l'aloe-émodine.

5. Utilisation selon l'une quelconque des revendication 1 à 4, **caractérisée en ce que** l'extrait de sauge contenu dans l'association est un extrait de feuilles de sauge.

6. Utilisation selon l'une quelconque des revendication 1 à 5, **caractérisée en ce que** l'extrait de feuilles de sauge contenu dans l'association contient au moins 1,5 % de tanins, par rapport à la matière sèche.

7. Utilisation selon l'une quelconque des revendication 1 à 6, **caractérisée en ce que** les tanins de l'extrait de feuilles de sauge contenu dans l'association contiennent de l'acide rosmarinique.

8. Utilisation selon l'une quelconque des revendication 1 à 7, pour le traitement local de manifestations qui ont été causées par des virus dépourvus d'enveloppe.

9. Utilisation selon l'une quelconque des revendication 1 à 8, pour le traitement local de manifestations provoquées par des infections à Herpès virus.

10. Utilisation selon l'une quelconque des revendication 1 à 9, pour le traitement local de manifestations provoquées par des infections à virus Herpès simplex 1 et 2.

11. Utilisation selon l'une quelconque des revendication 1 à 10, dans laquelle la composition se trouve sous forme d'une crème ou d'un bâton.

12. Utilisation selon l'une quelconque des revendication 1 à 11, **caractérisée en ce que** les extraits contenus dans l'association sont contenus en un rapport de mélange de 1:1.